# DEMANDE DE BREVET EUROPEEN

(11) **EP 3 760 113 A1**
(43) Date de publication de la demande: **06.01.2021**
(21) Numéro de dépôt: 20183491.8
(22) Date de dépôt: 01.07.2020
(51) Int. Cl.: A61B 5/042, A61N 1/05, A61N 1/375

(54) **SONDE SOUS CUTANÉE POUR UN DISPOSITIF CARDIAQUE IMPLANTABLE**

(30) Priorité: 05.07.2019 FR 1907535
(71) Demandeur: Sorin CRM SAS, 92140 Clamart (FR)
(72) Inventeur: OLLIVIER, Jean-Francois, 91190 Gif sur Yvette (FR)
(74) Mandataire: Grünecker Patent- und Rechtsanwälte PartG mbB

(57) **Abrégé**

La présente invention se rapporte à une sonde sous cutanée (10, 100) pour un dispositif cardiaque implantable (14), en particulier pour un défibrillateur ou/et un stimulateur cardiaque, comprenant un corps de sonde (20, 102), lui-même comprenant au moins une électrode de détection (16, 108, 110, 112); et comprenant en outre un manchon isolant (22, 118) dans lequel le corps de sonde (20, 102) est enfilé de manière à ce que le manchon isolant (22, 118) et le corps de sonde (20, 102) sont déplaçables l'un par rapport à l'autre de manière à couvrir au moins partiellement l'électrode de détection (16, 108, 110, 112) par le manchon isolant (22, 118). Le corps de sonde (20, 102) et le manchon isolant (22, 118) étant déplaçables l'un par rapport à l'autre, le positionnement longitudinal du manchon isolant (22, 118) par rapport au corps de sonde (20, 102) est ajustable par le médecin lors de l'implantation de la sonde (10, 100).

## Description

La présente invention se rapporte à une sonde sous cutanée pour un dispositif cardiaque implantable, en particulier pour un défibrillateur ou/et un stimulateur cardiaque.

Dans l'exemple d'un défibrillateur implantable, une sonde sous cutanée a une double fonction: elle permet, au moyen d'électrodes de détection, de détecter une activité électrique de surface pour en déduire d'activité cardiaque d'un patient et, si besoin, de délivrer un choc de défibrillation.

Cependant, la qualité de la détection d'une activité électrique de surface (donc distante de la masse cardiaque) peut être altérée par de nombreux artefacts tels que des bruits électriques musculaires de surface, des interférences avec le milieu extérieur etc. De plus, elle dépend largement du positionnement des électrodes de détection. De ce fait, le médecin doit trouver, lors de l'implantation de la sonde sous cutanée, un compromis de positionnement qui soit acceptable pour assurer la double fonction de la sonde ; au détriment parfois, selon la morphologie du patient, de la fonction de détection ou de défibrillation. Ceci est notamment dû au fait que les sondes sous-cutanées connues sont à géométrie fixe, c'est-à-dire que le positionnement relatif des électrodes est fixe par rapport au corps de sonde et ne peuvent pas prendre en compte les différentes morphologies des patients.

En outre, la distance au muscle excitable et la configuration des sondes sous-cutanées connues (notamment la distance inter-électrodes et le fait d'utiliser des électrodes de détection annulaires) ne sont pas adaptées pour créer un champ électrique distant suffisamment efficace sur une partie du muscle cardiaque qui puisse permettre une fonction de stimulation cardiaque (« pacing » en anglais).

La présente invention a ainsi pour objet de proposer une sonde sous cutanée pour un dispositif cardiaque implantable qui permette d'améliorer la qualité de la détection d'une activité électrique de surface. En outre, l'invention a pour objet de proposer une sonde sous cutanée adaptée pour la délivrance d'impulsions électriques au muscle cardiaque.

L'objet de la présente invention est atteint par une sonde sous cutanée pour un dispositif cardiaque implantable, en particulier pour un défibrillateur ou/et un stimulateur cardiaque, comprenant un corps de sonde lui-même comprenant au moins une électrode de détection ; et comprenant en outre un manchon isolant dans lequel le corps de sonde est enfilé de manière à ce que le manchon isolant et le corps de sonde sont déplaçables l'un par rapport à l'autre de manière à couvrir au moins partiellement l'électrode de détection par le manchon isolant.

Ainsi, de par son caractère déplaçable, le manchon isolant selon la présente invention permet de s'acquitter des limitations induites par la configuration fixe de l'électrode de détection par rapport au corps de sonde. De ce fait, le manchon isolant de la présente invention permet à un médecin de disposer d'une sonde sous-cutanée avec une électrode de détection à géométrie variable, rendant ainsi possible de s'adapter au mieux à la morphologie d'un patient. En effet, le corps de sonde et le manchon isolant étant déplaçables l'un par rapport à l'autre, le positionnement longitudinal du manchon isolant par rapport au corps de sonde est ajustable par le médecin lors de l'implantation de la sonde. La sonde sous-cutanée à géométrie variable de la présente invention est ainsi adaptée pour minimiser la détection de bruit musculaire de surface et donc pour éviter la détection d'artefact pouvant conduire à la délivrance de choc inapproprié.

La présente invention, relative à une sonde sous cutanée pour un dispositif cardiaque implantable, peut être davantage améliorée grâce aux modes de réalisation suivants.

Selon un mode de réalisation de l'invention, le manchon isolant peut avoir une longueur le long d'un axe central du manchon isolant plus longue qu'une longueur de l'électrode de détection le long d'un axe central de l'électrode de détection; et le manchon isolant peut comprendre une ouverture latérale dans une paroi latérale du manchon isolant de sorte que l'ouverture latérale peut être positionnée au-dessus de l'électrode de détection.

L'ouverture latérale du manchon isolant permet d'exposer sélectivement au moins partiellement une surface de l'électrode de détection. Le positionnement du manchon isolant par rapport à l'électrode de détection et au corps de sonde permet ainsi d'ajuster à la fois le positionnement de l'électrode de détection par rapport au corps de sonde, mais aussi la surface de l'électrode de détection exposée. Ainsi, grâce au manchon isolant rendant les paramètres de positionnement et de surface d'exposition de l'électrode de détection ajustables, la sonde sous-cutanée de la présente invention permet d'affiner le positionnement de l'électrode de détection et d'améliorer la détection d'une activité cardiaque d'un patient.

Selon un mode de réalisation de l'invention, le manchon isolant, respectivement le corps de sonde, peut être déplaçable longitudinalement et/ou radialement par rapport à un axe central du corps de sonde, respectivement à un axe central du manchon isolant.

De ce fait, le manchon isolant de la présente invention permet en outre, en plus des paramètres de positionnement et de surface d'exposition de l'électrode de détection ajustables, d'adapter sélectivement l'orientation de la surface exposée de l'électrode de détection, c'est-à-dire l'orientation de la surface de l'électrode de détection qui se trouve en-dessous de l'ouverture latérale du manchon isolant. Ainsi, la qualité de la détection des signaux électriques de surface peut être davantage améliorée par ce paramètre de réglage additionnel que constitue l'orientation de surface active (exposée) de l'électrode de détection.

En outre, l'orientation de la surface exposée de l'électrode de détection permet également, dans le cas d'une sonde pour un dispositif de stimulation cardiaque, d'orienter un champ électrique vers la masse cardiaque du patient.

Selon un mode de réalisation de l'invention, le corps de sonde peut en outre comprendre une deuxième électrode de détection; le manchon isolant peut couvrir au moins partiellement la première électrode de détection et la deuxième électrode de détection.

Ainsi, dans le cas d'une sonde sous-cutanée comprenant deux électrodes de détection, le manchon isolant permet aussi à un médecin de faire varier la surface respective exposée des deux électrodes de détection. En positionnant le manchon isolant par rapport à la première et à la deuxième électrodes de détection de manière adaptée à la morphologie d'un patient, la qualité de la détection des signaux électriques de surface par les deux électrodes de détection peut ainsi être améliorée.

Selon un mode de réalisation de l'invention, le manchon isolant peut comprendre deux ouvertures latérales dans une paroi latérale du manchon isolant de sorte qu'une première ouverture latérale peut être positionnée au-dessus de la première électrode de détection et une deuxième ouverture latérale peut être positionnée au-dessus de la deuxième électrode de détection.

Il est alors possible de définir préférentiellement, lors de la conception du manchon isolant, la distance entre les deux ouvertures latérales et ainsi de contrôler la distance entre les surfaces respectives exposées des électrodes de détection, qui forment un dipôle. De plus, il est possible de déplacer le long du corps de sonde la position du dipôle formé par les deux électrodes de détection au moyen du manchon isolant et, ainsi d'ajuster au mieux son positionnement (par rapport à deux extrémités opposées du corps de sonde) en fonction de la qualité de détection des signaux électriques de surface.

Selon un mode de réalisation de l'invention, la sonde peut en outre comprendre une troisième électrode de détection disposée d'une manière distale sur le corps de sonde ; et une électrode de défibrillation ; l'électrode de défibrillation peut être positionnée entre la troisième électrode de détection et un ensemble formé par la première électrode et la deuxième électrode de détection; le manchon isolant pouvant recouvrir au moins partiellement la première et la deuxième électrodes de détection.

La sonde sous-cutanée selon la présente invention est ainsi configurée pour un défibrillateur. Le manchon isolant permet de dissocier géométriquement la fonction de détection (des électrodes de détection) de la fonction de défibrillation (de l'électrode de défibrillation). En effet, le déplacement du manchon isolant sur le corps de sonde permet d'éviter que l'optimisation de la détection des signaux électriques de surface par la première et la deuxième électrodes de détection ne se fasse au détriment de la position de l'électrode de défibrillation.

Selon un mode de réalisation de l'invention, l'ensemble formé par la première et la deuxième électrodes de détection peut être configuré pour une fonction de stimulation cardiaque.

Ainsi la sonde sous-cutanée de la présente invention est également adaptée pour un dispositif de stimulation cardiaque de manière à ce que l'ensemble formé par la première et la deuxième électrode de détection soit capable de créer un champ électrique et que ce champ électrique soit orienté vers la masse cardiaque d'un patient au moyen du manchon isolant.

Selon un mode de réalisation de l'invention, une première portion du manchon isolant selon un axe central du manchon isolant peut comprendre une paroi latérale qui comprend elle-même au moins une surface plane, en particulier une portion du manchon isolant selon un axe central du manchon isolant peut avoir une section transversale par rapport à un axe central du manchon isolant de géométrie essentiellement polygonale, de préférence triangulaire.

La surface plane de la paroi latérale du manchon isolant offre une surface d'appui au manchon isolant avec les tissus musculaires d'un patient. La surface plane permet alors de maintenir, en particulier par friction, l'orientation du manchon isolant par rapport au corps de sonde une fois implanté dans le corps d'un patient, en évitant un mouvement involontaire en rotation du manchon isolant autour du corps de sonde, lors de l'implantation ou au cours de vie du patient implanté.

La géométrie essentiellement polygone, de préférence triangulaire, d'une portion du manchon isolant permet d'améliorer davantage le maintien de l'orientation du manchon isolant par rapport au corps de sonde dans le corps d'un patient, en offrant davantage d'appui entre ladite portion du manchon isolant et les tissus musculaires d'un patient.

Selon un mode de réalisation de l'invention, une deuxième portion du manchon isolant selon un axe central du manchon isolant peut comprendre au moins un moyen de fixation configuré pour empêcher un déplacement longitudinal du manchon isolant par rapport à un axe central du corps de sonde et ou pour attacher le manchon isolant à un tissue musculaire.

Le moyen de fixation permet d'assurer le maintien du manchon isolant dans sa position optimale définie par le médecin après l'implantation de la sonde sous-cutanée. Le positionnement ajusté de l'électrode de détection peut ainsi être maintenu et assuré lors de la vie du patient implanté.

Selon un mode de réalisation de l'invention, le corps de sonde peut comprendre au moins un élément de butée faisant saillie depuis le corps de sonde au moins partiellement autour du corps de sonde, apte à bloquer un déplacement longitudinal du manchon isolant par rapport à un axe central du corps de sonde.

L'élément en butée du corps de sonde permet ainsi de contrôler l'amplitude du déplacement longitudinal du manchon isolant par rapport à un axe central du corps de sonde.

Selon un mode de réalisation de l'invention, au moins une électrode de détection est une électrode flexible comprenant un ou plusieurs filaments enroulé(s) autour du corps de sonde, en particulier au moins une électrode parmi la première électrode de détection et la deuxième électrode de détection de la sonde. L'avantage des électrodes flexibles est qu'elles permettent d'éviter de rigidifier le corps de sonde, en comparaison avec des électrodes rigides classiques, en particulier de longues électrodes avec sous-couche en silicone, ce qui contribue à éviter de fragiliser la sonde dans le temps et/ou être inconfortable pour le patient.

La présente invention permet ainsi d'éviter l'utilisation de sonde classique comprenant des anneaux de détection rigides.

Selon un mode de réalisation de l'invention, le manchon isolant peut être réalisé en au moins deux parties distinctes, chaque partie du manchon isolant comprenant au moins une ouverture latérale.

Il est ainsi possible d'adapter la conception de la sonde sous-cutanée de la présente invention a plusieurs jeux de manchon isolant afin de s'ajuster au mieux aux besoins du patient et du médecin, notamment en faisant varier la distance inter-électrodes. De plus, les au moins deux parties distinctes du manchon isolant peuvent être dotées d'ouvertures latérales de différentes dimensions. Il est aussi possible de proposer des ouvertures différentes spécifiquement adaptées aux besoins du patient et du médecin.

Selon un mode de réalisation de l'invention, le manchon isolant peut comprendre un matériau radio opaque.

Ainsi, le manchon isolant a l'avantage d'être radiodétectable et peut donc être identifiable lors d'une radiographie médicale.

L'invention et ses avantages seront expliqués plus en détail dans la suite au moyen de modes de réalisation préférés et en s'appuyant notamment sur les figures d'accompagnement suivantes, dans lesquelles :
Figure 1 représente une vue schématisée et par transparence d'une sonde sous cutanée selon un premier mode de réalisation de la présente invention ;
Figure 2 représente un agrandissement de la Figure 1 de la sonde sous cutanée selon le premier mode de réalisation ;
Figure 3 représente une vue schématisée et par transparence d'une sonde sous cutanée selon un deuxième mode de réalisation de la présente invention ;

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

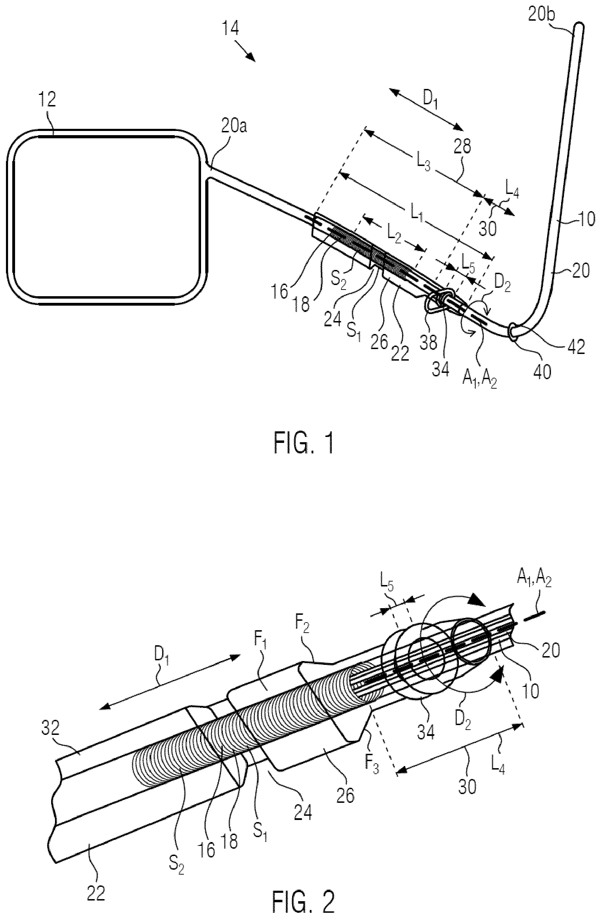

La Figure 1 illustre une sonde sous cutanée 10, selon un premier mode de réalisation, connectée à un boîtier 12 d'un dispositif médical 14, qui est implanté par voie sous-cutanée.

La Figure 2 étant un agrandissement d'une section de la sonde sous cutanée 10, les Figures 1 et 2 seront décrites conjointement dans ce qui suit.

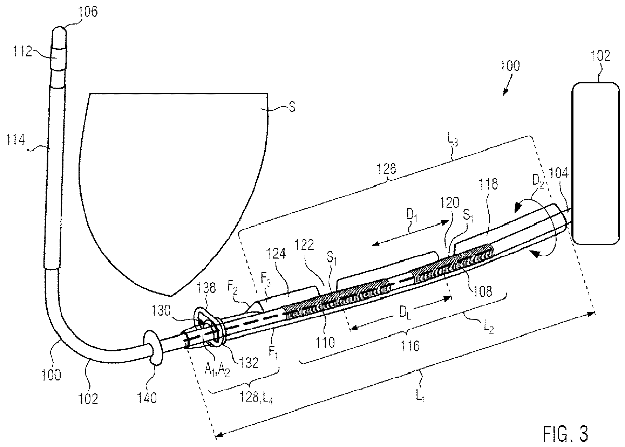

Selon le premier mode de réalisation, la sonde 10 comprend une électrode de détection 16. Dans une variante où le dispositif médical 14 est un défibrillateur implantable 14, la sonde sous cutanée comprend en outre une électrode de défibrillation. Un tel dispositif sera davantage décrit en référence à la Figure 3.

L'électrode de détection 16 est formée d'un mono-filament 18 enroulé autour du corps de sonde 20, visible sur l'agrandissement de la Figure 2. L'électrode de détection 16 est ainsi une électrode flexible contrairement aux sondes classiques connues de l'art antérieur qui sont dotées d'anneaux de détection rigides. L'électrode de détection 16 peut être partiellement enrobée d'une sous-couche de silicone ou de polyuréthane.

Le corps de sonde 20 est délimité par deux extrémités 20a, 20b dont l'une (20a) est connectée au boîtier 12 et l'autre (20b) correspond à l'extrémité distale de la sonde 10, visible sur la Figure 1.

L'électrode de détection 16 de la sonde sous-cutanée 10 permet la détection de signaux électriques de surface dont le traitement permet d'en déduire l'activité cardiaque d'un patient.

La détection d'activité électrique en surface est cependant altérée par de nombreux artefacts comme des bruits électriques musculaires de surface ou des interférences avec le milieu extérieur. La qualité de la détection dépend ainsi largement du positionnement de l'électrode de détection 16 dans le corps du patient.

Afin de contrôler le positionnement de l'électrode de détection 16 pour améliorer la qualité de la détection en surface, la sonde sous-cutanée 10 de la présente invention comprend un manchon isolant 22 dans lequel le corps de sonde 20 est enfilé. Le manchon isolant 22 peut être réalisé en polyuréthane afin de lui conférer une certaine rigidité en torsion. Dans une variante, le manchon isolant 22 peut être réalisé en silicone biocompatible (50 ou 65 Shore). Dans une autre variante, le manchon isolant 22 comprend en outre un matériau radio opaque, comme du sulfate de baryum, afin de rendre le manchon isolant 22 détectable lors d'une radiographie médicale.

Le corps de sonde 20 et le manchon isolant 22 sont déplaçables l'un par rapport à l'autre de manière à couvrir au moins partiellement l'électrode de détection 16 par le manchon isolant 22. Le manchon isolant 22 est ainsi déplaçable longitudinalement (représenté par la double flèche D1) et/ou radialement (représenté par la double flèche D2) par rapport à un axe central A2 du corps de sonde 20. Respectivement, le corps de sonde 20 est déplaçable longitudinalement (représenté par la double flèche D1) et/ou radialement (représenté par la double flèche D2) par rapport à un axe central A1 du manchon isolant 22.

Ainsi, l'ajustement de la position du manchon isolant 22 permet de s'acquitter des limitations induites par la configuration fixe de l'électrode de détection 16 par rapport au corps de sonde 20. De ce fait, le manchon isolant 22 permet à un médecin de disposer d'une sonde sous-cutanée 10 avec une électrode de détection 16 à géométrie variable, rendant ainsi possible de s'adapter au mieux à la morphologie d'un patient et de pouvoir réduire voire minimiser la détection de bruit musculaire de surface (et donc d'éviter la détection d'artefact pouvant conduire à la délivrance de choc inapproprié par exemple).

Le manchon isolant 22 a une longueur L1 le long d'un axe central A1 du manchon isolant 22 plus longue qu'une longueur L2 de l'électrode de détection 16 le long d'un axe centrale A2 (voir Figure 1). De plus, le manchon isolant 22 comprend une ouverture latérale 24, telle qu'une fenêtre, dans une paroi latérale 26 du manchon isolant 22 de sorte que l'ouverture latérale 24 est positionnée au-dessus de l'électrode de détection 16.

La superficie totale S2 de l'électrode de détection 16 est comprise entre 200 millimètres carré et 500 millimètres carré. Le manchon isolant 22 permet de réduire la superficie totale S2 de l'électrode de détection 16. L'ouverture latérale 24 du manchon isolant 22 permet ainsi d'exposer sélectivement au moins partiellement une surface S1 de l'électrode de détection 16, c'est-à-dire la surface S1 de l'électrode de détection 16 qui n'est pas recouverte par le manchon isolant 22. La superficie de la surface S1 exposée de l'électrode de détection 16 est donc inférieure à la superficie de la surface totale S2 de l'électrode de détection 16. Selon le premier mode de réalisation, la surface S1 exposée de l'électrode de détection 16 a une superficie de 4 à 30 millimètres carré, de préférence de 10 à 20 millimètres carré. La superficie de la surface exposée S1 peut être préférentiellement définie lors de la conception du manchon isolant 22 tandis que le positionnement de l'ouverture latérale 24 au-dessus de l'électrode de détection 16 est contrôlé par le médecin lors de l'implantation de la sonde sous cutanée 10. Cela confère une géométrie variable à la sonde sous-cutanée 10, ce qui permet d'améliorer la qualité de la détection en surface grâce à un positionnement ajusté de l'électrode de détection 16. Ainsi la distance de la partie découverte par rapport aux extrémités 20a, 20b peut être réglée par le médecin.

Dans une variante, une paroi latérale 26 du manchon isolant 22 peut être pourvue d'au moins deux ouvertures latérales. De plus, la géométrie de l'ouverture latérale 24 peut varier d'un manchon isolant 22 à un autre, et peut, en particulier, être spécifiquement conçu pour s'adapter aux besoins de détection ou/et de stimulation propre(s) à un patient. La forme spécifique de l'ouverture latérale 24 du manchon isolant 22 selon le premier mode de réalisation illustrée aux Figures 1 et 2 n'est donc pas limitative et pourrait être conçue avec une géométrie différente - du moins tant que l'ouverture latérale 24 permet d'exposer une surface S1 de l'électrode de détection 16 qui soit inférieure à la surface totale S2 de l'électrode de détection 16.

L'ouverture latérale 24 du manchon isolant 22 permet d'obtenir une électrode de détection 16 de type sectoriel et non annulaire. Le caractère sectoriel de l'ouverture latérale 24 du manchon isolant 22 permet notamment d'orienter préférentiellement la surface exposée de l'électrode de détection vers la masse cardiaque. L'orientation préférentielle de la surface exposée S1 de l'électrode de détection 16, en particulier vers la masse musculaire du patient implanté, permet notamment de minimiser la détection de bruit musculaire de surface et donc potentiellement d'éviter la détection d'artefacts pouvant conduire à des chocs inappropriés.

Selon le premier mode de réalisation, le manchon isolant 22 est formé d'une seule pièce qui comprend selon un axe central A1 une première portion 28 de longueur L3 et une deuxième portion 30 de longueur L4. Selon le premier mode de réalisation, la première portion 28 du manchon isolant 22 a une section transversale essentiellement polygonale, ici triangulaire, tandis que la deuxième portion 30 du manchon isolant 22 a une section transversale essentiellement circulaire.

La première portion 28 du manchon isolant 22 est ainsi pourvue d'une paroi latérale 32 selon axe central A1 du manchon isolant 22 qui comprend essentiellement trois faces planes F1, F2, F3 (voir Figure 2).

Dans une variante, la première portion 28 du manchon isolant 22 n'a pas nécessairement une section de géométrie triangulaire mais comprend une paroi latérale selon un axe central A1 du manchon isolant 22 comprenant elle-même au moins une surface plane.

Les faces planes F1, F2, F3 de la paroi latérale 32 de la première portion 28 du manchon isolant 22 fournissent chacune une surface d'appui au manchon isolant 22 avec des tissus musculaires d'un patient. Les faces planes F1, F2, F3 permettent alors de maintenir, en particulier par friction, l'orientation du manchon isolant 22 par rapport au corps de sonde 20 dans le corps d'un patient, en évitant un mouvement involontaire en rotation (représenté par la double flèche D2) du manchon isolant 22 autour du corps de sonde 20, lors de l'implantation et au cours de la vie du patient implanté.

La deuxième portion 30 du manchon isolant 22, dont la section transversale est essentiellement circulaire, comprend quant à elle un moyen de fixation 34 permettant à un médecin d'immobiliser le manchon isolant 22 par rapport au corps de sonde 20, et d'attacher l'ensemble manchon isolant 22 et corps de sonde 20 aux tissus musculaires d'un patient.

Selon le premier mode de réalisation de l'invention, le moyen de fixation 34 est fourni par une rainure 36 sur la deuxième portion 30 qui est transversale à un axe central A1 du manchon isolant 22. La rainure 36 a une largeur L5 adaptée pour recevoir un fil de ligature 38. Ainsi, une fois le positionnement défini par le médecin, en fonction de la qualité des signaux électriques détectés par exemple, le manchon isolant 22 et le corps de sonde 20 sont alors solidarisés au niveau du thorax d'un patient par une technique classique de ligature, le fil de ligature 38 englobant à la fois le manchon isolant 22 et le corps de sonde 20 au niveau de la rainure 36, et les tissus musculaires du patient.

La Figure 1 illustre en outre un élément de butée 40 disposé sur le corps de sonde 20 qui permette de contrôler l'amplitude du déplacement longitudinal (représenté par la flèche D1) du manchon isolant 22 par rapport au corps de sonde 20 en bloquant le déplacement longitudinal (représenté par la flèche D1) du manchon isolant 22 au-delà de l'élément de butée 40 (respectivement en-deçà - selon la position du manchon isolant 22 par rapport à l'élément de butée 40).

Selon le premier mode de réalisation, l'élément de butée 40 est une protubérance en forme d'anneau 42 qui s'étend depuis le corps de sonde 20 et autour du corps de sonde 20 et qui fait donc saillie depuis le corps de sonde 20. L'élément de butée 40 n'est pas limité à une géométrie annulaire et peut prendre une forme de protubérance différente tant que l'élément de butée soit dimensionné de manière à bloquer un déplacement longitudinal du manchon isolant 22. Dans une variante, le corps de sonde peut comprendre deux éléments de butée qui sont positionnées de manière à ce que le manchon isolant 22 soit capable d'être déplacé longitudinalement entre les deux éléments de butée.

La Figure 3 représente une vue schématisée et par transparence d'une sonde sous cutanée 100 selon un deuxième mode de réalisation de la présente invention. En comparaison avec le premier mode de réalisation, la sonde sous-cutanée 100 du deuxième mode de réalisation est connectée à un boîtier 102 d'un dispositif médical de type défibrillateur, qui est implanté par voie sous-cutanée (le sternum d'un patient est indiqué par la référence « S » à la Figure 3). La sonde sous-cutanée 100 du deuxième mode de réalisation est ainsi configurée à la fois pour une fonction de détection et une fonction de défibrillation.

La sonde 100 a un corps de sonde 102 délimité par deux extrémités 104, 106 ; l'extrémité 104 étant connectée au boîtier 102 (c'est l'extrémité proximale de la sonde 100) et l'extrémité 106 correspondant à l'extrémité distale de la sonde 100.

Selon le deuxième mode de réalisation, la sonde 100 comprend une première électrode de détection 108, une deuxième électrode de détection 110 et une troisième électrode de détection 112. La sonde 100 comprend en outre une électrode de défibrillation 114. Selon la configuration du deuxième mode de réalisation, la première électrode de détection 108 est placée de manière proximale sur le corps de sonde 102 tandis que la troisième électrode de détection 112 est placée au niveau de l'extrémité distale 106 du corps de sonde 102. La deuxième électrode de détection 110 est placée entre la première électrode de détection 108 et la troisième électrode de détection 112. L'électrode de défibrillation 114 est quant à elle placée entre la deuxième électrode de détection 108 la troisième électrode de détection 112.

De la même manière que l'électrode de détection 16 du premier mode de réalisation et de ses variantes, les électrodes de détection 108, 110, 112 sont formées d'un mono-filament ou de plusieurs filaments enroulé(s) autour du corps de sonde 102.

Les électrodes de détection 108, 110, 112 de la sonde sous-cutanée 100 permettent la détection de signaux électriques de surface dont le traitement permet d'en déduire l'activité cardiaque d'un patient tandis que l'électrode de défibrillation 114 a une fonction de défibrillation. Le médecin a ainsi besoin d'ajuster le positionnement de la sonde 100 en veillant à la fois à un fonctionnement optimal de la fonction de détection et de la fonction de défibrillation. Afin d'ajuster en particulier le positionnement du dipôle 116 formé par la première électrode de détection 108 et la deuxième électrode de détection 110 pour améliorer la qualité de la détection en surface, la sonde sous-cutanée 100 de la présente invention comprend un manchon isolant 118 dans lequel le corps de sonde 102 est enfilé et qui couvre au moins partiellement la première électrode de détection 108 et la deuxième électrode de détection 110.

Comme décrit par rapport au manchon isolant 22 du premier mode de réalisation, le manchon isolant 118 selon le deuxième mode de réalisation est déplaçable par rapport au corps de sonde 102. Le manchon isolant 118 est ainsi déplaçable longitudinalement (représenté par la double flèche D1) et radialement (représenté par la double flèche D2) par rapport à un axe central A2 du corps de sonde 102. Respectivement, le corps de sonde 102 est déplaçable longitudinalement (représenté par la double flèche D1) et radialement (représenté par la double flèche D2) par rapport à un axe central A1 du manchon isolant 118.

En comparaison avec le manchon isolant 22 du premier mode de réalisation, le manchon isolant 118 a une longueur L1 le long d'un axe central A1 du manchon isolant 118 plus longue qu'une longueur L2 du dipôle 116 le long d'un d'axe centrale A2 du corps de sonde 102. De plus, le manchon isolant 118 selon le deuxième mode de réalisation comprend deux ouvertures latérales 120, 122 dans une paroi latérale 124 du manchon isolant 118 de sorte qu'une première ouverture latérale 120, telle qu'une fenêtre, est positionnée au-dessus de la première électrode de détection 108 et une deuxième ouverture latérale 122, telle qu'une fenêtre, est positionnée au-dessus de la deuxième électrode de détection 110.

Selon le deuxième mode de réalisation, la première ouverture latérale 120 et la deuxième ouverture latérale 122 ont une géométrie identique. Cependant, dans une variante, la première et la deuxième ouvertures latérales 120, 122 pourraient être de forme différente l'une par rapport à l'autre. La géométrie de chaque ouverture latérale 120, 122 peut ainsi varier et être spécifiquement conçu pour s'adapter aux besoins de détection et/ou aux besoin thérapeutiques du patient.

Ainsi, l'ajustement de la position du manchon isolant 118 permet de s'acquitter des limitations induites par la configuration fixe entre autre, des électrodes de détection 108, 110 du dipôle 116 par rapport au corps de sonde 102. De ce fait, le manchon isolant 118 permet à un médecin de disposer d'une sonde sous-cutanée 100 avec un dipôle 116 d'électrodes de détection 108, 110 à géométrie variable, rendant ainsi possible de s'adapter au mieux à la morphologie d'un patient et de pouvoir minimiser la détection de bruit musculaire de surface et donc d'éviter la détection d'artefact pouvant conduire à la délivrance de choc inapproprié par exemple. Ainsi la distance du dipôle par rapport à l'électrode de défibrillation 114 et la troisième électrode 112 peut être ajustée par le médecin lors de l'implantation.

La première ouverture latérale 120 et la deuxième ouverture latérale 122 du manchon isolant 118 permettent ainsi d'exposer sélectivement au moins partiellement une surface respective S1 de la première électrode de détection 108 et de la deuxième électrode de détection 110, c'est-à-dire la surface S1 de chaque électrode de détection 108, 110 qui n'est pas recouverte par le manchon isolant 118. La superficie de chaque surface exposée S1 peut être préférentiellement définie lors de la conception du manchon isolant 118, de même que la distance longitudinale DL entre la première ouverture latérale 120 et la deuxième ouverture latérale 122 par rapport à un axe central A1 du manchon isolant 118. La détermination de ces paramètres (S1, DL) de géométrie du manchon isolant 118 permet de configurer le dipôle 116 formé par la première électrode de détection 108 et la deuxième électrode de détection 110. Cela confère une géométrie variable à la sonde sous-cutanée 100, ce qui permet d'améliorer la qualité de la détection en surface grâce à un positionnement ajusté du dipôle 116. Le médecin peut en outre, lors de l'implantation de la sonde 100, adapter le positionnement des ouvertures latérales 120, 122 au-dessus de la première et de la deuxième électrode de détection 108, 110 afin d'optimiser le positionnement du dipôle 116, notamment par rapport à la troisième électrode de détection distale 112.

De plus, comme décrit dans le premier mode de réalisation, les ouvertures latérales 120, 122 du manchon isolant 118 permettent d'obtenir des électrodes de détection 108, 110 de type sectoriel et non annulaire. Le caractère sectoriel des ouvertures latérales 120, 122 du manchon isolant 118 permettent notamment d'orienter préférentiellement la surface exposée S1 des électrodes de détection 120, 122 vers la masse cardiaque. L'orientation préférentielle des surface exposée S1 du dipôle 116, en particulier vers la masse musculaire du patient implanté, permet notamment de minimiser la détection de bruit musculaire de surface et donc potentiellement d'éviter la détection d'artefacts pouvant conduire à des chocs inappropriés.

De plus, dans le deuxième mode de réalisation, l'ajustement de l'orientation d'un champ électrique créé depuis le dipôle 116 de la sonde 100 vers une masse cardiaque du patient peut être utilisée pour une fonction de stimulation cardiaque ; par exemple, en combinaison avec une fonction de défibrillation.

Comme dans le premier mode de réalisation, le manchon isolant 118 est formé d'une seule pièce qui comprend selon un axe central A1 une première portion 126 de longueur L3 et une deuxième portion 128 de longueur L4. La première portion 126 du manchon isolant 118 a une section transversale essentiellement polygonale, ici triangulaire, tandis que la deuxième portion 128 du manchon isolant 118 a une section transversale essentiellement circulaire.

La première portion 126 du manchon isolant 118 est ainsi pourvue d'une paroi latérale 124 selon axe central A1 du manchon isolant 118 qui comprend essentiellement trois faces planes F1, F2, F3.

Dans une variante, la première portion 126 du manchon isolant 118 n'a pas nécessairement une section de géométrie triangulaire mais comprend une paroi latérale selon un axe central A1 du manchon isolant 118 comprenant elle-même au moins une surface plane.

Les faces planes F1, F2, F3 de la paroi latérale 124 de la première portion 126 du manchon isolant 118 fournissent chacune une surface d'appui au manchon isolant 118 avec des tissus musculaires d'un patient. Les faces planes F1, F2, F3 permettent alors de maintenir, en particulier par friction, l'orientation du manchon isolant 118 par rapport au corps de sonde 102 dans le corps d'un patient, en évitant un mouvement involontaire en rotation (représenté par la double flèche D2) du manchon isolant 118 autour du corps de sonde 102, lors de l'implantation et au cours de la vie du patient implanté.

La deuxième portion 108 du manchon isolant 118, dont la section transversale est essentiellement circulaire, comprend quant à elle un moyen de fixation 130 permettant à un médecin d'immobiliser le manchon isolant 118 au corps de sonde 102, et d'attacher l'ensemble manchon isolant 118 et corps de sonde 102 aux tissus musculaires d'un patient. Comme dans le premier mode de réalisation de l'invention, le moyen de fixation 130 est fourni par une rainure 132 sur la deuxième portion 108 qui est transversale à un axe central A1 du manchon isolant 118. La rainure 136 est adaptée pour recevoir un fil de ligature 138. Ainsi, une fois le positionnement défini par le médecin, en fonction de la qualité des signaux électriques détectés par exemple, le manchon isolant 118 et le corps de sonde 102 sont alors solidarisés sur le thorax d'un patient par une technique classique de ligature, le fil de ligature 138 englobant à la fois le manchon isolant 118 et le corps de sonde 102 au niveau de la rainure 38, et les tissus musculaires du patient.

Le manchon isolant 118 étant ainsi fixé via le fil de ligature 138 aux tissus musculaire du patient, dans le cas d'un déplacement involontaire de la sonde 100 dans le corps du patient, celui-ci n'aurait pas d'impact sur la configuration du dipôle 116 lui-même car la position et l'orientation des surfaces exposées S1 du dipôle 116 sont assurées par la fixation entre le manchon isolant 118 et les tissus musculaire du patient (et donc, dans une certaine mesure, indépendamment de la position de la sonde 100). D'autant plus que le manchon isolant 118 peut aussi comprendre au moins un élément de butée 140, tel que décrit dans le premier mode de réalisation, disposé sur le corps de sonde 102 qui permette de contrôler l'amplitude du déplacement longitudinal (représenté par la flèche D1) du manchon isolant 118 par rapport au corps de sonde 102 en bloquant le déplacement longitudinal (représenté par la flèche D1) du manchon isolant 118 au-delà de l'élément de butée 140 (respectivement en-deçà - selon la position du manchon isolant 118 par rapport à l'élément de butée 40).

Les modes de réalisation décrits sont simplement des configurations possibles et il faut garder à l'esprit que les caractéristiques individuelles du premier mode de réalisation et du deuxième mode de réalisation peuvent être combinées entre elles ou fournies indépendamment les unes des autres.

## Revendications

1. Sonde sous cutanée pour un dispositif cardiaque implantable, en particulier pour un défibrillateur ou/et un stimulateur cardiaque (14),
comprenant un corps de sonde (20), lui-même comprenant au moins une électrode de détection (16); et
comprenant en outre un manchon isolant (22) dans lequel le corps de sonde (20) est enfilé de manière à ce que le manchon isolant (22) et le corps de sonde (20) sont déplaçables l'un par rapport à l'autre de manière à couvrir au moins partiellement l'électrode de détection (16) par le manchon isolant (22).

2. La sonde sous cutanée selon la revendication 1, dont le manchon isolant (22) a une longueur (L1) le long d'un axe central (A1) du manchon isolant (22) plus longue qu'une longueur (L2) de l'électrode de détection (16) le long d'un d'axe central (A2) de l'électrode de détection (16); et
dont le manchon isolant (22) comprend une ouverture latérale (24) dans une paroi latérale (26) du manchon isolant (22) de sorte que l'ouverture latérale (24) est positionnée au-dessus de l'électrode de détection (16).

3. La sonde sous cutanée selon la revendication 1 ou 2, dont le manchon isolant (22, 118), respectivement le corps de sonde (20, 102), est déplaçable longitudinalement et/ou radialement par rapport à un axe central du corps de sonde (20, 102), respectivement à un axe central (A1) du manchon isolant (22, 118).

4. La sonde sous cutanée selon l'une des revendications précédentes, dont le corps de sonde (102) comprend en outre une deuxième électrode de détection (110); et
dont le manchon isolant (118) couvre au moins partiellement la première électrode de détection (108) et la deuxième électrode de détection (110).

5. La sonde sous cutanée selon la revendication 4, dont le manchon isolant (118) comprend deux ouvertures latérales (120, 122) dans une paroi latérale (124) du manchon isolant (118) de sorte qu'une première ouverture latérale (120) est positionnée au-dessus de la première électrode de détection (108) et une deuxième ouverture latérale (122) est positionnée au-dessus de la deuxième électrode de détection (110).

6. La sonde sous cutanée selon la revendication 4 ou 5, comprenant en outre une troisième électrode de détection (112) disposée d'une manière distale sur le corps de sonde (102); et
une électrode de défibrillation (114);
telle que l'électrode de défibrillation (114) est positionnée entre la troisième électrode de détection (112) et un ensemble formé par la première et la deuxième électrodes de détection (108, 110);
le manchon isolant (118) recouvrant au moins partiellement la première électrode de détection (108) et la deuxième électrode de détection (110).

7. La sonde sous cutanée selon la revendication 4, 5 ou 6, dont l'ensemble formé par la première et la deuxième électrodes de détection (108, 110) est configuré pour une fonction de stimulation cardiaque.

8. La sonde sous cutanée selon l'une des revendication précédentes, dont une première portion (126) du manchon isolant (118) selon un axe central (A1) du manchon isolant (118) comprend une paroi latérale (124) qui comprend elle-même au moins une surface plane, en particulier une portion (126) du manchon isolant (118) selon un axe central du manchon isolant (118) a une section transversale par rapport à un axe central du manchon isolant (118) de géométrie essentiellement polygonale, de préférence triangulaire.

9. La sonde sous cutanée selon la revendication 8, dont une deuxième portion (30, 128) du manchon isolant (22, 118) selon un axe central (A1) du manchon isolant (22, 118) comprend au moins un moyen de fixation (34, 130) configuré pour empêcher un déplacement longitudinal du manchon isolant (22, 118) par rapport à un axe central du corps de sonde (20, 102) et/ou pour attacher le manchon isolant (22, 118) à un tissue musculaire.

10. La sonde sous cutanée selon l'une des revendications précédentes, dont le corps de sonde (20, 102) comprend au moins un élément de butée (40, 140) faisant saillie depuis le corps de sonde (20, 102) au moins partiellement autour du corps de sonde (20, 102) apte à bloquer un déplacement longitudinal du manchon isolant (22, 118) par rapport à un axe central du corps de sonde (20, 102).

11. La sonde sous cutanée selon l'une des revendications précédentes, dont au moins une électrode de détection (16, 108, 110, 112) est une électrode flexible comprenant un ou plusieurs filaments enroulé(s) autour du corps de sonde (20, 102), en particulier au moins une électrode parmi la première électrode de détection (108) et la deuxième électrode de détection (110) selon la revendication 4.

12. La sonde sous cutanée selon l'une des revendications précédentes, dont le manchon isolant (22, 108) est réalisé en au moins deux parties distinctes, chaque partie du manchon isolant comprenant au moins une ouverture latérale (24, 120, 122).

13. La sonde sous cutanée selon l'une des revendications précédentes, dont le manchon isolant (22, 108) comprend un matériau radio opaque.
